(19) Europäisches Patentamt
European Patent Office
Office européen des brevets

(11) Veröffentlichungsnummer : **0 380 010 B1**

(12) # EUROPÄISCHE PATENTSCHRIFT

(45) Veröffentlichungstag der Patentschrift : **30.11.94 Patentblatt 94/48**

(51) Int. Cl.$^5$ : **G03F 7/029**

(21) Anmeldenummer : **90101157.7**

(22) Anmeldetag : **20.01.90**

(54) **Strahlungsempfindliche, ethylenisch ungesättigte, copolymerisierbare Sulfoniumsalze und Verfahren zu deren Herstellung.**

(30) Priorität : **25.01.89 DE 3902114**

(43) Veröffentlichungstag der Anmeldung : **01.08.90 Patentblatt 90/31**

(45) Bekanntmachung des Hinweises auf die Patenterteilung : **30.11.94 Patentblatt 94/48**

(84) Benannte Vertragsstaaten : **BE CH DE FR GB IT LI NL SE**

(56) Entgegenhaltungen :
**EP-A- 0 035 969**
**DE-A- 3 604 581**

(73) Patentinhaber : **BASF Aktiengesellschaft**
**Carl-Bosch-Strasse 38**
**D-67063 Ludwigshafen (DE)**

(72) Erfinder : **Boettcher, Andreas, Dr.**
**Konrad-Adenauer-Ring 38**
**D-6907 Nussloch (DE)**

EP 0 380 010 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neuartige strahlungsempfindliche, ethylenisch ungesättigte Sulfoniumsalze und ein Verfahren zu ihrer Herstellung.

Sulfoniumsalze werden zur Härtung kationisch polymerisierbarer Monomere eingesetzt. Früher härtete man kationisch polymerisierbare Monomere, wie z.B. Epoxide, mit sauren Katalysatoren, wie in der US-PS 38 42 019 beschrieben. Beispielsweise kann man Epoxide mit Bortrifluorid und seinen Komplexen härten, während man Styrol mit Aluminiumtrifluorid polymerisieren kann. Außerdem kann man 1,4-Diazabicyclo[2.2.2]-octan mit Benzolsulfonsäure polymerisieren. Während das Härten solcher Monomerer mit solchen Katalysatoren erfolgreiche Ergebnisse in vielen Fällen bewirkt, sind saure Katalysatoren oft unerwünscht, weil derartige Katalysatoren verschiedene Substrate, wie z.B. Metalle, stark korrodieren. Außerdem haben viele dieser sauren Katalysatoren keine zufriedenstellende Haltbarkeit, sobald sie dem zu polymerisierenden Material zugemischt sind. Ferner sind Katalysatoren des Stands der Technik, wie z.B. $BF_3 \cdot NH_2C_2H_5$ feuchtigkeitsempfindlich.

Gemäß US-PS 38 42 019 verwendet man bestimmte Sulfonsäuresalze, die ausschließlich thermisch aktiviert werden. Jedoch erfordern diese Gemische Härtungstemperaturen von 150 bis 200°C. Derartige härtbare Mischungen sind zur Herstellung von hitzeempfindlichen elektronischen Bauteilen ungeeignet. In besonderen Fällen kann das kationische Härten verschiedener Zusammensetzungen erleichtert werden, wenn man ein besonders lichtempfindliches Sulfonsäuresalz, wie z.B. das entsprechende Silbersalz, als Katalysator verwendet. Die Verwendung derartiger Sulfonsäure-Metallsalz-Zusammensetzungen ist jedoch auf spezielle Anwendungen beschränkt.

In der DE-A-25 18 652 sind härtbare Zusammensetzungen beschrieben, die ein polymerisierbares Epoxyharz und ein strahlungsempfindliches Oniumsalz, wie Triphenylsulfoniumhexafluoroantimonat, enthalten.

Oniumsalze werden weiterhin u.a. in der Mikrolithographie zur photochemischen Löslichkeitsdifferenzierung eingesetzt. Die in der DE-A-27 54 853 beschriebenen speziellen Oniumsalze spalten bei Belichtung eine starke, nicht nucleophile Säure ab, die zur Löslichkeitsdifferenzierung von Photopolymeren dient (vgl. z.B. Polym. Eng. Sci. 23, 953 (1983)), indem eine säurelabile Schutzgruppe, z.B. die tert.-Butoxycarbonyloxy-Gruppe (vgl. Polymer 24 995 (1983); Macromolecules 16, 510 (1983); Polym. Eng. Sci. 23, 1022 (1983)) durch die erzeugte Säure abgespalten wird.

Auch in der DE-A-37 21 740 sind Sulfoniumsalze, die mindestens eine durch Säure spaltbare Gruppierung enthalten beschrieben.

Die Oniumsalze werden den Monomer/Polymermischungen zugesetzt (vgl. Chim. Nuov. 4, 343 (1986)). Im allgemeinen sind solche Arbeitsweisen aber nicht vollständig befriedigend, denn es treten nach Mischung mit dem Polymeren Probleme mit der Verträglichkeit, der Löslichkeit, der Gleichförmigkeit, der Verteilung, der Flüchtigkeit, des Geruches, der Toxizität, des Ausschwitzens und der Wanderung des Zusatzstoffes auf, die häufig zu einer unerwünschten, vorzeitigen und ungleichmäßigen Reaktion führen. Beim eigentlichen Belichtungsvorgang wird dann eine geringe Reaktivität aufgrund erniedrigter effektiver Initiatorkonzentration sowie nach dem Belichten eine Reihe störender Nebenreaktionen beobachtet.

Für die Herstellung von Halbleiter-Photoresisten werden Sulfoniumsalze häufig als Initiatoren verwendet. Beim Auftragen der in einem organischen Lösungsmittel gelösten Oligomermischungen auf einen Träger beobachtet man häufig (vgl. Macromolecules 16, 510 (1983)) beim Abdampfen des Lösungsmittels eine Anreicherung der polaren Sulfoniumsalze in den unteren Bereichen der polymerschicht, in denen das Lösungsmittel am längsten verbleibt. Daraus resultiert eine Photopolymerschicht mit einem Initiatorkonzentrationsgradienten, der zu schlechten, ungleichmäßigen Härtungsergebnissen führt.

Von der Chemie anderer photoinitiatorklassen ist bekannt, daß die genannten Probleme z.T. gelöst werden können, wenn der Strahlungsempfindliche Initiator mit Monomeren nach einem üblichen Verfahren mischpolymerisiert, d.h. in eine Polymerkette eingebaut wird. Der lichtempfindliche photoinitiator hängt mit einer Ankergruppe, dem sog. Spacer, am Basispolymeren. Der Spacer dient weiterhin dazu, den Einfluß der Basispolymerkette auf das photochemische Verhalten des Initiators zu reduzieren.

Copolymerisierbare Initiatoren haben daher prinzipiell folgenden Aufbau:

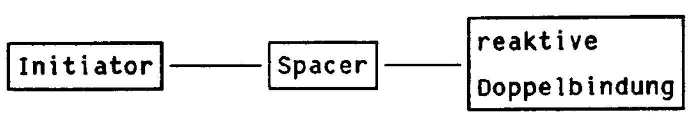


Schema I

Eine Reihe von polymergebundenen Sulfoniumsalzen, wie z.B.

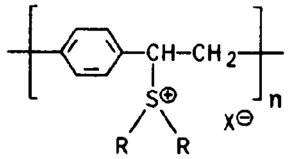

(vgl. Polymer 27, 1709 (1986)),

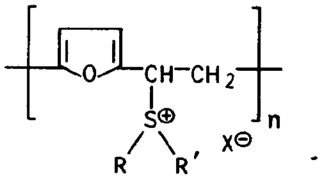

(vgl. EP-A-246 931),

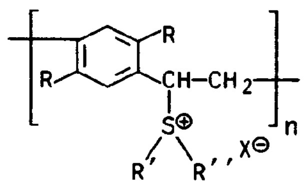

(vgl. EP-A-246 931, Polym. Commun. 26, 362 (1985))
worin
R, R′, R″ = Alkyl
X = Halogen
n ≧ 5 sind,
sind bereits beschrieben worden.

Sulfoniumsalze mit ethylenisch ungesättigten, reaktiven Gruppen werden als latente thermische Katalysatoren

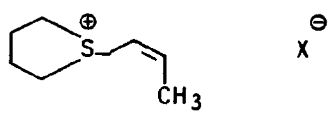

(vgl. Makromol. Chem., Rapid Commun. 6, 137 (1985))
oder als copolymerisierbare Monomere eingesetzt:

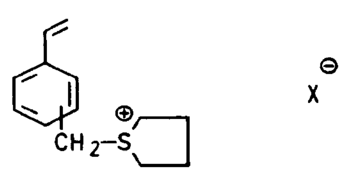

(vgl. J. Polym. Sci., Part C, Polym. Lett. 26, 77 (1988)).

Alle bisher bekannten, polymer gebundenen Sulfoniumsalze sind weniger toxisch und bilden bei der Photolyse weniger flüchtige Nebenprodukte als die entsprechenden monomeren Oniumsalze.

Allerdings sind die oben erwähnten Sulfoniumsalze photochemisch weniger reaktiv, da der Spacer sehr kurz ist und z.T. völlig fehlt. Dadurch besitzt die Oniumgruppe nur eine geringe konformative Beweglichkeit.

Aufgabe der vorliegenden Erfindung ist es, neue copolymerisierbare Sulfoniumsalze der Typen

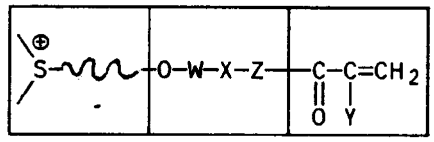

Initiator Spacer reaktive Gruppe

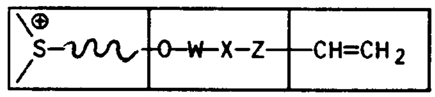

Initiator Spacer reaktive Gruppe

aufzuzeigen, die die oben genannten Nachteile nicht aufweisen und besonders migrationsstabil sind.

Gegenstand der vorliegenden Erfindung sind strahlungsempfindliche, ethylenisch ungesättigte, copolymerisierbare, organische Verbindungen der allgemeinen Formel (I)

$$[(R)_a(R^1)_b(R^2)_c \overset{\oplus}{S}]A^{\ominus} \quad (I),$$

wobei

R einen, gegebenenfalls substituierten, einwertigen aromatischen organischen Rest bedeutet,

$R^1$ einen, gegebenenfalls substituierten, einwertigen organischen aliphatischen Rest aus der Gruppe der Alkyl-, Cycloalkyl- und substituierten Alkylreste bedeutet,

$R^2$ einen, gegebenenfalls substituierten, zwei- oder dreiwertigen aliphatischen oder aromatischen organischen Rest bedeutet, der eine heterocyclische oder kondensierte Ringstruktur bildet,

a eine ganze Zahl von 0 bis einschließlich 3 ist,

b eine ganze Zahl von 0 bis einschließlich 2 ist,

c eine ganze Zahl gleich 0 oder 1 ist,

wobei die Summe a+b+c=3 ist und

$A^{\ominus}$ für das Anion einer Säure steht

und mindestens einer der Reste R bis $R^2$ einen der Reste

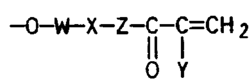

oder

$$-O-W-X-Z-CH=CH_2$$

gebunden enthält, worin

W für eine Einfachbindung oder eine der Gruppen

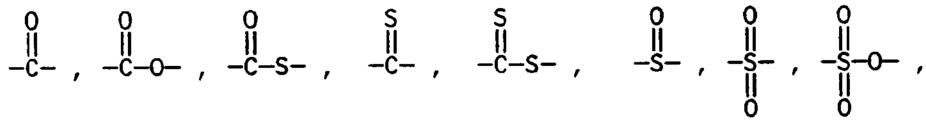

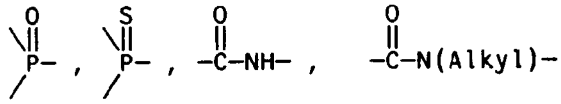

mit Alkyl, beispielsweise Methyl, Ethyl, n-Propyl, i-Propyl, n-Butyl,

X für einen zweiwertigen, gegebenenfalls substituierten, Alkylenrest $-(CH_2)_m-$, einen Rest

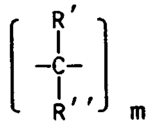 ,

mit m = 1 bis 10,

worin R′ und R″ untereinander gleich oder verschieden sind und für Aryl, z.B. Phenyl, $C_1$- bis $C_4$-Alkyl, H, COOH, $COOCH_3$ oder $COOC_2H_5$ stehen, einen perfluorierten Alkylenrest $-(CF_2)_m-$ mit m = 1 bis 10, vorzugsweise einen Perfluorethylenrest beispielsweise einen Tetrafluorethylen, einen Oxaalkylenrest des Typs $-(CH_2)_n-O-(CH_2)_p-$ mit n = 1 bis 5 und p = 1 bis 5, vorzugsweise n = p = 2, d.h. $-C_2H_4-O-C_2H_4-$, einen perfluorierten Oxaalkylenrest des Typs $-(CF_2)_n-O-(CF_2)_p-$ mit n, p = 1 bis 5, oder einen gegebenenfalls perfluorierten Polyoxaalkylenrest mit 2 bis 20 Sauerstoffatomen, die miteinander über mindestens eine $-CH_2-$, $-CF_2-$oder $-CH_2-CH(CH_3)-$Gruppe verbunden sind, für einen Alkylenrest des Typs $-(CH_2)_m-O-CO-O-(CH_2)_n-$, $-(CH_2)_n-O-CO-NH-(CH_2)_m-$, $-(CH_2)_n-NH-CO-O-(CH_2)_m-$, $-(CH_2)_m-CO-O-(CH_2)_n-$oder $-(CH_2)_m-O-CO-(CH_2)_n-$ mit m = 1 bis 10, n = 1 bis 10, einen gegebenenfalls mit Alkyl mit 1 bis 4 Kohlenstoffatomen, z.B. Methyl, Ethyl, n-Propyl, iso-Propyl, OH, $OCH_3$, $OC_2H_5$, SH, $SCH_3$, $SC_2H_5$, Cl, F, $N(Alkyl)_2$ oder $N(CH_3)C_6H_5$ in o-, m- und/oder p-Stellung substituierten Phenylen-, oder einen Cycloalkylenrest mit 5 bis 10 Kohlenstoff-Atomen, z.B. Cyclohexylen-, Cyclooctylen- oder für einen (Bis)methylencycloalkylenrest mit 6 bis 12 Kohlenstoffatomen steht,

Y für H, Alkyl mit 1 bis 6 Kohlenstoffatomen oder Phenyl, und

Z für O, NY stehen.

Beispiele für R in der allgemeinen Formel (I) sind, gegebenenfalls substituierte, $C_{(6-13)}$ aromatische Kohlenwasserstoffreste, wie Phenyl, Tolyl, 4-(Phenylthio)phenyl, Naphthyl, Anthryl usw. sowie solche mit 1 bis 4 einwertigen Resten substituierte aromatische Kohlenwasserstoffreste, wobei die Substituenten solche sein können, wie $C_{(1-8)}$-Alkoxy, $C_{(1-8)}$-Alkyl, Nitro, Chlor, Hydroxy usw., weiter können für R Arylalkylreste, wie Benzyl, Phenacylreste, aromatische heterocyclische Reste, wie Pyridyl, Furfuryl usw. stehen.

Die Reste $R^1$ schließen ein $C_{(1-8)}$ Alkyl, wie Methyl, Ethyl usw., substiuiertes Alkyl, wie $-C_2H_4OCH_3$, $-CH_2COOC_2H_5$, $-CH_2COCH_3$ usw. ein.

Die Reste $R^2$ schließen solche Strukturen ein, wie

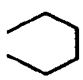 , 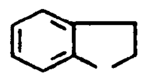 , 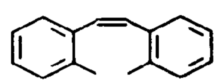 , 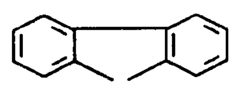 ,

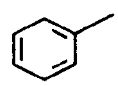

Unter den Anionen $A^{\ominus}$ der Formel (I) sind z.B. $BF_4^{\ominus}$, $PF_6^{\ominus}$, $AsF_6^{\ominus}$, $SbF_6^{\ominus}$, $ClO_4^{\ominus}$, $CF_3SO_3^{\ominus}$, $AlCl_4^{\ominus}$, $BCl_4^{\ominus}$, $Br^{\ominus}$, $Cl^{\ominus}$, $HSO_4^{\ominus}$, $CH_3CO_2^{\ominus}$, $NO_3^{\ominus}$ usw. sowie $(MSO_3)^{\ominus}$ zu verstehen, wobei M aus der Gruppe der aromatischen $C_1$ bis $C_{13}$-Kohlenwasserstoffreste, $C_1$ bis $C_8$-Alkylreste und ihrer halogenierten Derivate ausgewählt ist.

Überraschenderweise zeigen die erfindungsgemäßen Verbindungen je nach Substitutionsmuster eine besonders hohe photochemische Reaktivität sowohl im kurzwelligen UV-Bereich von 250 - 350 nm, als auch im längerwelligen Bereich von 330 bis 430 µm.

Eine weitere Aufgabe der vorliegenden Erfindung bestand darin, ein Verfahren zur Herstellung neuer, strahlungsempfindlicher, copolymerisierbarer Sulfoniumsalze mit mindestens einer Acrylat- oder Vinyletherendgruppe aufzuzeigen.

Diese Aufgabe wurde gelöst, indem hydroxygruppenhaltige Sulfoniumsalze mit Isocyanaten oder Chlorformiaten bzw. deren thermisch stabilen Vorstufen ggf. in Gegenwart eines Katalysators umgesetzt werden.

Überraschenderweise wurde gefunden, daß die erfindungsgemäßen Sulfoniumsalze leicht und in sehr guter Ausbeute zugänglich sind. Dies ist insbesondere im Hinblick auf die Reaktivität und Bifunktionalität der (Meth)acrylat-, Vinylether- und Isocyanatkomponente unerwartet, da eine Vielzahl verschiedener Reaktionsprodukte denkbar ist.

Gegenstand der vorliegenden Erfindung ist außerdem ein Verfahren zur Herstellung von Verbindungen der allgemeinen Formel (I), wobei man eine Verbindung der Formel (II), (III) oder (IV)

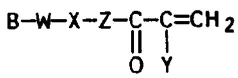 , $B-W-X-Z-CH=CH_2$ oder 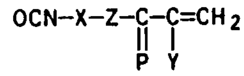

(II) (III) (IV)

worin

W, X, Y und Z die oben genannte Bedeutung haben und

B für eine der Gruppen Tosylat, Alkoxy mit 1 bis 5 Kohlenstoffatomen, Halogen, wie z.B. Cl, Br, Chlorcarbonyl, Imidazolyl, Pyrazolyl, Ammonium-, Pyridinium-, Phosphonium- oder Sulfoniumkation steht - vorzugsweise wären dies z.B. 2-(Acryloyloxyethyl)-, 2-(Methacryloyloxyethyl)chlorkohlensäureester, 2-(Methacryloyloxyethyl)chlorglyoxylsäureester, (2-(Meth)acryloyloxyethyl)-methyl-carbonat - mit einer Verbindung der allgemeinen Formel (V)

$$[(R)_a(R^1)_b(R^2)_c \overset{\oplus}{S}]A^{\ominus} \quad (V),$$

wobei

R einen, gegebenenfalls substituierten einwertigen aromatischen organischen Rest bedeutet,

$R^1$ einen gegebenenfalls substituierten einwertigen organischen aliphatischen Rest aus der Gruppe der Alkyl-, Cycloalkyl- und substituierten Alkylreste bedeutet,

$R^2$ einen gegebenenfalls substituierten zwei- oder dreiwertigen aliphatischen oder aromatischen organischen Rest bedeutet, der eine heterocyclische oder kondensierte Ringstruktur bildet,

a eine ganze Zahl von 0 bis einschließlich 3 ist,

b eine ganze Zahl von 0 bis einschließlich 2 ist,

c eine ganze Zahl gleich 0 oder 1 ist,
wobei die Summe a+b+c=3 ist und

$A^{\ominus}$ für das Anion einer Säure steht

und mindestens einer der Reste R bis $R^2$ eine Hydroxylgruppe enthält, beispielsweise

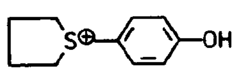 $BF_4^{\ominus}$

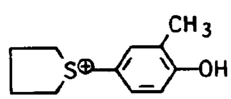 $PF_6^{\ominus}$

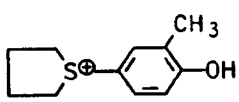 $BF_4^{\ominus}$

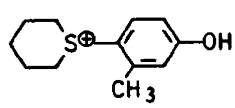 $Br^{\ominus}$

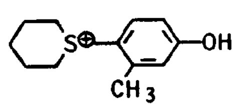 $BF_4^{\ominus}$

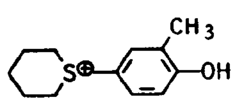 $PF_6^{\ominus}$

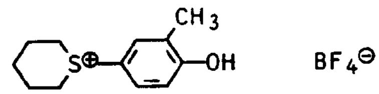

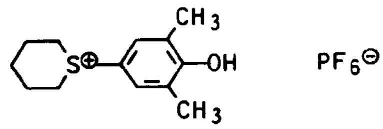

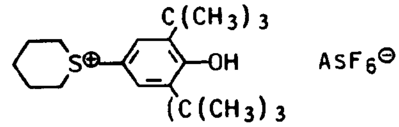

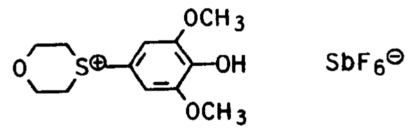

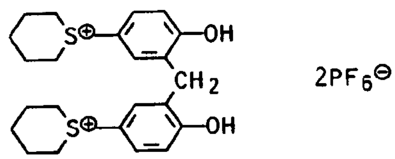

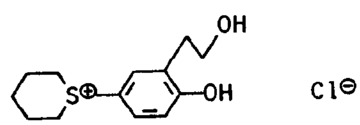

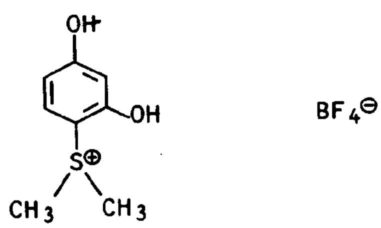

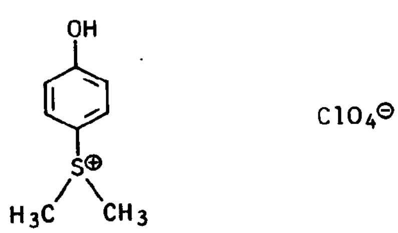

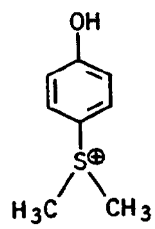
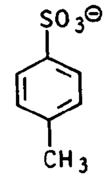

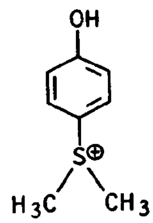

$H_3CSO_3^{\ominus}$

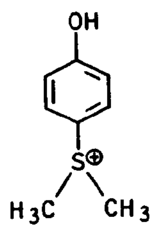

$HSO_4^{\ominus}$

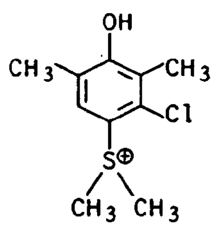

$AsF_6^{\ominus}$

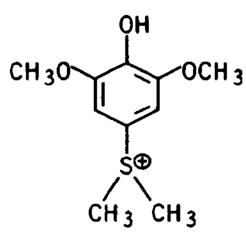

$AsF_6^{\ominus}$

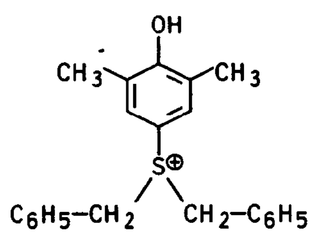

$PF_6^{\ominus}$

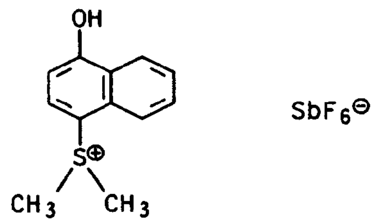

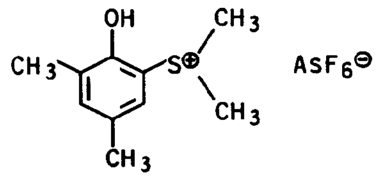

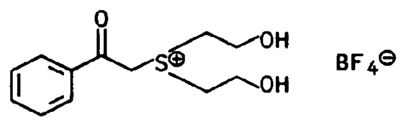

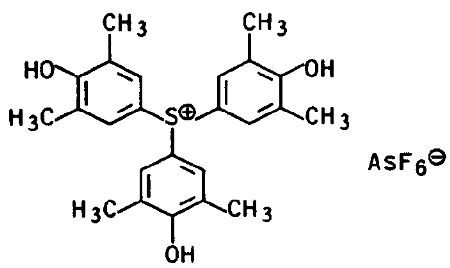

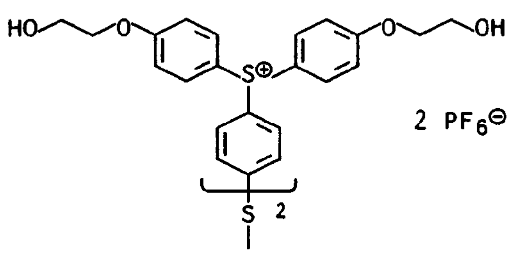

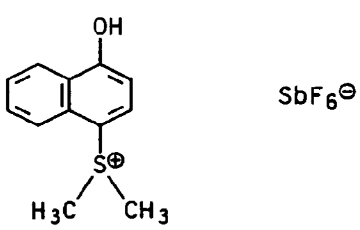

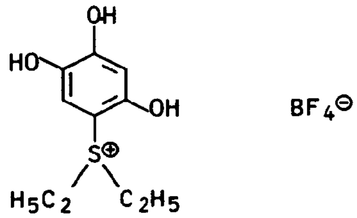

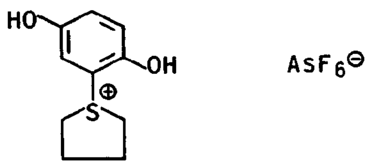

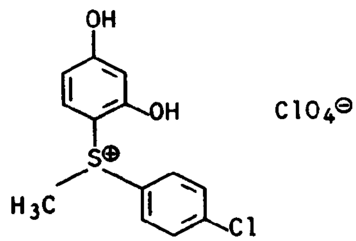

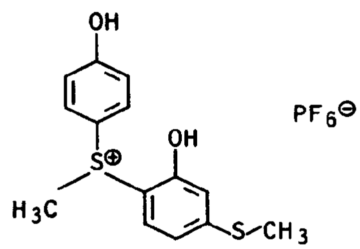

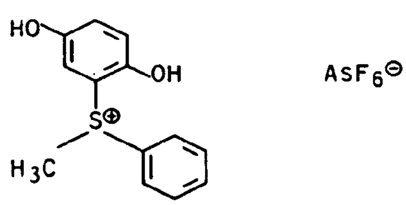

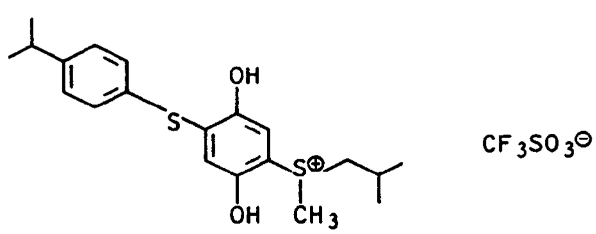

im äquimolaren Verhältnis (gegebenenfalls mit bis zu 10 bis 30 %igem Überschuß) oder entsprechend der Anzahl der Hydroxylgruppen in den Resten R bis $R^2$ dem Zwei- oder Dreifachen davon, gegebenenfalls in Gegenwart eines inerten Lösungsmittels oder Lösungsmittelgemisches und eines basischen Katalysators, bei

Temperaturen von 0 bis 100°C, vorzugsweise bei 10 bis 60°C, umsetzt.

Einige bei der Reaktion eingesetzte Chlorformiate und die Isocyanate reagieren leicht mit Nucleophilen, u.a. auch mit Wasser. Deshalb ist bei der Reaktion auf Feuchtigkeitsausschluß durch Verwendung getrockneter und/oder schwach bzw. nicht nucleophiler Lösungsmittel, wie z.B. Acetonitril, Dichlormethan, Dichlorethan, Tetrahydrofuran, Toluol, Xylol, Chlorbenzol, Essigester, Chloroform usw. und gegebenenfalls auf den Aufbau einer Inertgasatmosphäre, z.B. Stickstoff, Argon oder Kohlendioxid zu achten.

Die Synthesen der als Ausgangsstoffe notwendigen Hydroxysulfoniumsalze sind bekannt. Beispielhaft seien die folgenden Literaturstellen genannt:
J. Am. Chem. Soc. 80, 3425 (1958);
J. Polym. Sci. Polym. Chem. Ed. 18, 1021 (1980);
Polym. Prep. Am. Chem. Soc. Div. Polym. Chem. 25, 262 (1984);
Polym. J. 17, 73 (1985); US-P 4 336 363; US-P 4 417 061; US-P 4 650 734;
US-P 4 684 671; EP 245 662; J-PS 61 212 555; DE-OS 1 951 803;
DE-PS 25 41 709 usw.
und die hierin zitierte Literatur.

Die ω-(Meth)acryloyloxyalkyl-chlorformiate sind nach literaturbekannten verfahren, wie z.B. in Eur. Polym. J. 14, 205 (1978); 3. Polym. Sci. Polym. Symp. 66, 41 (1979); Bull. Soc. Chim. Belg. 93, 159 (1984) beschrieben, bequem und in guten Ausbeuten darstellbar.

Beispiele für Verbindungen dieses Typs sind:

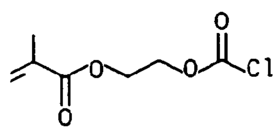

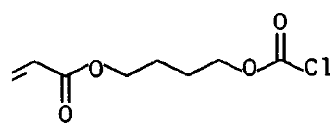

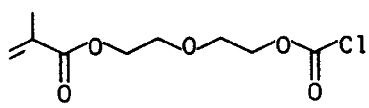

Andere Methoden, zur Herstellung der Chlorformiate, die sich insbesondere für Laborsynthesen eignen, ergeben sich durch die Auswahl der Phosgenierungsmittel. Als Phosgen-Alternativen seien das Trichlormethylchlorformiat (Diphosgen), J. Prakt. Chem. 126, 210 (1930), dito 128, 233 (1930), Chem. Abstr. 95, 81766, J. Org. Chem. 50, 715 (1985), J. Org. Chem. 41, 2070 (1976), Angew. Chem. 89, 267 (1977), das kristalline Triphosgen, Angew. Chem. 99, 922 (1987), das N,N'-Carbonyldiimidazol oder das N,N'-Carbonyldi-s-triazol (Fieser 1, 116 (1967)) genannt.

Mit Hilfe dieser Reagenzien sind beispielsweise darstellbar:

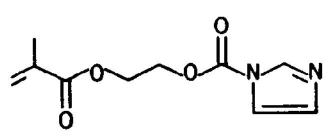

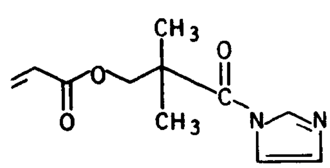

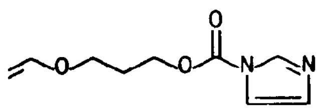

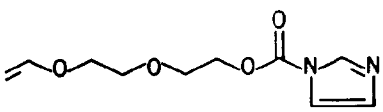

Über den Einsatz weiterer spezieller alternativer Verfahren für die Phosgenierung, z.B. die Umsetzung mit chlorkohlensäureestern gibt "Merck Kontakte" 1981 (1), 1-18 Auskunft.

Die Umsetzung von p-Hydroxyphenylsulfoniumsalzen mit aktivierten Säurechloriden ist in EP-A-245 662 beschrieben. Dazu werden die folgenden Verbindungen

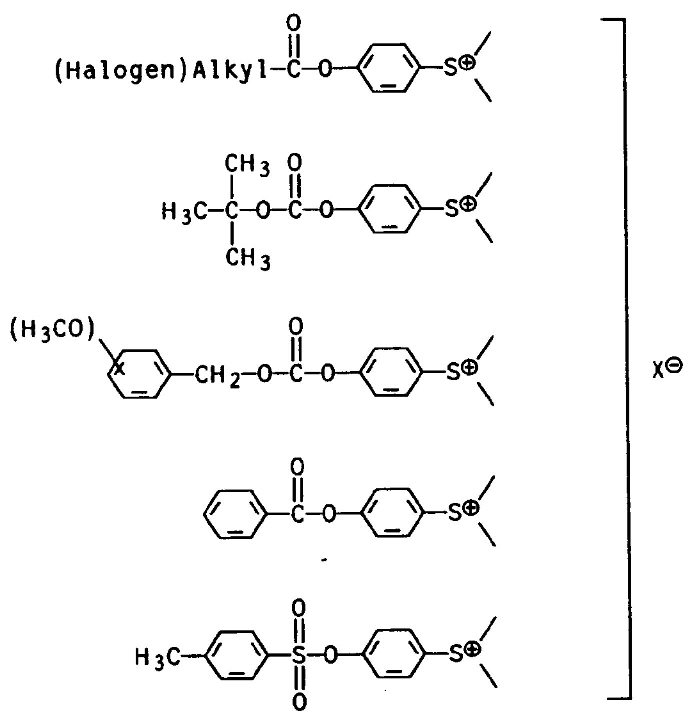

aus den entsprechenden Säurechloriden bzw. Chlorformiaten hergestellt. Die Sulfoniumsalze sind sehr reaktiv; sie werden als aktivierte Ester in der Peptidsynthese eingesetzt. Es ist nun überraschend, daß die Chlorformiate der Hydroxyalkyl(meth)acrylate gegenüber Nucleophilen stabil sind und z.T. sogar aus Isopropanol umkristallisiert werden können. Diese Eigenschaften machen sie besonders interessant für den Einsatz in Polymeren mit nucleophilen Gruppen.

Die ω-Isocyanatoalkyl(meth)acrylate können in guter Ausbeute nach den in den Patentschriften EP-A-083 764 und DE-A-35 23 692 beschriebenen Verfahren synthetisiert werden. Beispielhaft seien die folgenden Isocyanate genannt:

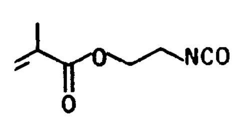

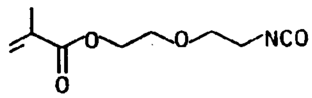

Zu anderen Isocyanaten gelangt man auf klassischem Wege, z.B. indem man nach dem Verfahren der US-PS 27 18 516 Alkanolamine mit Chlorameisensäurethylester umsetzt, das erhaltene Hydroxyalkylethylcarbonat mit Methacryloylchlorid acyliert und das dabei erhaltene Urethan in Gegenwart basischer Katalysatoren unter Erhitzen spaltet oder nach dem Verfahren der US-PS 28 21 544 auch durch Umsetzung von Methacryloylchlorid mit Alkanolaminhydrochloriden und anschließende Reaktion der erhaltenen ω-Aminoalkylmethacrylate mit Phosgen. Beispiele für geeignete Alkanolaminhydrochloride sind:

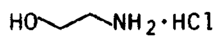

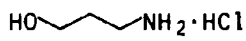

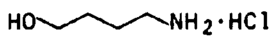

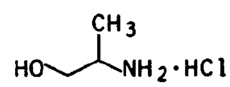

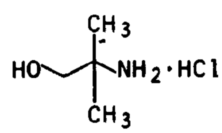

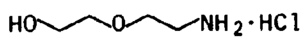

Für die Umsetzung der Hydroxy(aryl)sulfoniumsalze mit den Isocyanaten zu den entsprechenden Arylcarbamaten gibt es in der Literatur analoge Beispiele.

Die Synthese von Arylcarbamaten ohne copolymerisationsfähige Endgruppe ist bekannt. Eine Übersicht gibt C. Ferri, "Reaktionen der organischen Synthese", G. Thieme Verlag, Stuttgart, 1978.

Das wichtigste Darstellungsverfahren ist die Umsetzung von aromatischen Alkoholen mit Isocyanaten (vgl. Houben-Weyl VIII, S. 141; O.S. Petersen, Liebigs Ann. Chem. 562, 205 (1947); J. Burkus, J. Org. Chem. 26, 779 (1961); I.T. Kay und N. Punja, J. Chem. Soc. [C] 1968, 3011; L. Capuano und R. Zander, Chem. Ber. 104, 2212 (1971)). Die Carbamate entstehen in guten bis sehr guten Ausbeuten, wenn Alkohol und Isocyanat im Molverhältnis 1:1 ohne Solvens oder in überschüssigem Alkohol als Lösungsmittel miteinander zur Reaktion gebracht werden. In den Fällen, in denen der Alkohol bzw. das Phenol als Feststoff vorliegen, verwendet man aprotische Lösungsmittel, wie z.B. Dichlormethan, Dichlorethan, Acetonitril, Toluol usw.

Bei der Übertragung dieses Darstellungsverfahrens auf ω-Isocyanatoalkyl(meth)acrylate der allgemeinen Formel (IV), in der X für einen gegebenenfalls perfluorierten Alkylenrest, einen Oxaalkylenrest oder einen Polyoxaalkylenrest mit jeweils 2 bis 12 C-Atomen und Y für H- oder $CH_3$-stehen, wurde überraschend gefunden, daß die gewünschten Carbamoyl substituierten Sulfoniumsalze mit (Meth)Acrylatgruppen in hoher, fast quantitativer Ausbeute entstehen. Dies ist insofern überraschend, als Acrylate bzw. Methacrylate leicht zahlreiche Nebenreaktionen (Vernetzung, Polymerisation) eingehen können.

Überraschend sind weiterhin die guten bis hervorragenden Ausbeuten in Gegenwart der Sulfoniumgruppen.

Zum Herstellverfahren ist im einzelnen Folgendes auszuführen:

In der Regel wird eine Lösung oder Suspension der Hydroxyverbindung in einem inerten Lösungsmittel, welches auch wegfallen kann, wenn die Verbindung bei der Reaktionstemperatur flüssig ist, bei Temperaturen von 0 bis 100°C, vorzugsweise bei 10 bis 60°C, in Gegenwart eines basischen, schwach bzw. nicht nucleophilen Amins, vorzugsweise Triethylamin, 4-Dimethylaminopyridin, Imidazol, 1,4-Diazabicyclo[2.2.2]octan, 1,5-Diazabicyclo[4.3.0]non-5-en, 1,8-Diazabicyclo[5.4.0]undec-7-en, Polyvinylpyridin, N,N-Dimethylproyplenharnstoff, N,N'-Dimethylethylenharnstoff usw., vorgelegt. Dann wird beispielsweise die Chlorformylverbindung, u.U. gelöst in einem inerten Lösungsmittel, wie z.B. Dichlormethan, Dichlorethan,

Acetonitril, Toluol, Chlorbenzol, Xylol usw. unter Rühren im oben angegebenen Temperaturbereich zugetropft. Diese Arbeitsweise eignet sich besonders für größere Ansätze.

Nach einer Nachrührzeit von 1 bis 48 Stunden, vorzugsweise 1 bis 20 Stunden bei 10 bis 30°C wird nach Standardverfahren filtriert, gewaschen, getrocknet und das Produkt nach dem Umkristallisieren, Destillieren oder Extrahieren isoliert.

Die Erfindung betrifft auch kationisch härtbare Mischungen, die als Katalysator ein erfindungsgemäßes copolymerisierbares Sulfoniumsalz enthalten, durch Erhitzen oder Bestrahlen mit aktinischem Licht gehärtet werden können und sich zur Herstellung von Formkörpern, Beschichtungen, Reliefbildern und Resistmustern eignen.

Kationisch härtbare Mischungen, wie Epoxidharze werden üblicherweise mit Carbonsäuren oder deren Anhydriden oder durch Zusatz von anderen Lewis-Säuren gehärtet. Wegen der hohen Reaktivität müssen beide Komponenten getrennt gehandhabt und nach dem Vermischen rasch verarbeitet werden. Es hat nicht an Versuchen gefehlt, Einkomponentensysteme zu entwickeln, die längere Zeit lagerstabil sind und entweder durch Erhitzen oder durch Bestrahlung mit Licht geeigneter Wellenlänge gehärtet werden können. Als Katalysatoren für die durch Licht induzierte Härtung wurde eine Vielzahl von Photoinitiatoren beschrieben, unter denen vor allem die Diazoniumsalze der US-A 3 205 157 und US-A-3 708 296 sowie die Oniumsalze von Elementen der V. (siehe DE-A-2 518 656), VI. (siehe DE-A-2 518 652, DE-A - 904 626) und VII. (siehe DE-A-2 518 639) Hauptgruppen des Periodensystems der Elemente, sowie die in den EP-A-22 081, EP-A-35 969 und EP-A-44 274 genannten Sulfoxoniumsalze genannt seien. Diese Verbindungen sind jedoch in ihren Eigenschaften nicht befriedigend. Diazoniumsalze setzen bei der Bestrahlung Stickstoff frei, was zu einer Blasenbildung in mit Diazoniumsalzen hergestellten Beschichtungen und Formkörpern führen kann. Die Jodoniumsalze der DE-A-2 518 639 sind toxisch, ebenso wie die Sulfoniumsalze der DE-A-2 518 652 und DE-A-2 904 626 absorbieren sie zudem im Wellenlängenbereich zwischen 300 - 400 nm nur schwach, so daß den photohärtbaren Gemischen meist ein Sensibilisator zugesetzt werden muß. Ferner setzen manche der Sulfoniumsalze nach DE-A-2 518 652 und DE-A-2 904 626 bei der Bestrahlung mit aktinischem Licht übelriechende niedermolekulare Schwefelverbindungen frei. Die Sulfoxoniumsalze der EP-A-22 081, EP-A-35 969 und EP-A-44 274 sind nur umständlich unter Verwendung teurer metallorganischer Reagenzien zugänglich, was ihre Herstellung in technischen Mengen erschwert.

Als Katalysatoren für thermisch härtbare Systeme wurde in der DE-A 2 853 886 eine Kombination aus Jodonium-Salz und $Cu^I$-Salz beschrieben, die jedoch wegen des stark toxischen Jodonium-Salzes nur unter erheblichen Vorsichtsmaßnahmen eingesetzt werden kann. Eine weitere Katalysatorkombination ist die in der DE-A 3 135 636 beschriebene Mischung aus Pyryliumsalzen und Metallchelaten; jedoch läßt die Lagerstabilität der mit Hilfe dieser Initiatorkombination hergestellten Mischungen Wünsche offen.

Der Erfindung lag auch die Aufgabe zugrunde, kationisch härtbare Mischungen zu entwickeln, die einen kationisch wirksamen Härtungskatalysator enthalten, gut lagerfähig, leicht handhabbar, verarbeitungsfähig und nicht toxisch sind, und die nach der Härtung Formkörper mit guter Oberfläche und Lösungsmittelbeständigkeit ergeben.

Die erfindungsgemäßen härtbaren Zusammensetzungen enthalten z.B.

a) eine Verbindung bzw. ein Gemisch von Verbindungen, die unter dem Einfluß eines kationischen Katalysators in höhermolekulares Material überführbar sind und

b) bevorzugt 0,1 - 15 Gew.% der oben beschriebenen erfindungsgemäßen Sulfoniumsalze, bezogen auf die Menge der Verbindungen a).

Die Verbindungen a) können beispielsweise Oxetane, Thiirane oder Tetrahydrofuran sein. Vorzugsweise ist Verbindung a) ein 1,2-Epoxid, eine olefinisch ungesättigte Verbindung, ein Aminoplast oder ein Phenoplast, soweit diese kationisch härtbar bzw. polymerisierbar sind.

Beispiele geeigneter 1,2-Epoxide sind Epichlorhydrin, Propylenoxyd oder Glycidylether eines einwertigen Alkohols oder Phenols, wie n-Butylglycidylether oder Phenylglycidylether, Glycidylester wie Glycidylacrylat oder Glycidylmethacrylat. Vorzugsweise ist Komponente a) ein Epoxidharz und insbesondere ein solches, das mindestens eine direkt an ein Sauerstoffatom gebundene Gruppe der Formel (VI)

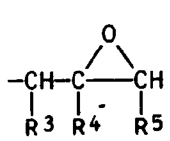

(VI)

enthält, worin entweder $R^3$ und $R^5$ je ein Wasserstoffatom darstellen, in welchem Fall $R^4$ ein Wasserstoffatom oder eine Methylgruppe bedeutet, oder $R^3$ und $R^5$ zusammen $-CH_2CH_2-$ darstellen, in welchem Fall $R^4$ ein Was-

serstoffatom bedeutet. Beispiele solcher Harze sind Polyglycidyl- und Poly(β-methylglycidyl)ester, die man durch Umsetzung einer zwei oder mehr Carbonsäuregruppen enthaltende Verbindung mit Epichlorhydrin, Glycerin-dichlorhydrin oder β-Methylepichlorhydrin in Gegenwart von Alkali erhalten kann. Solche Polyglycidylester können sich von aliphatischen Polycarbonsäuren, wie Bernsteinsäure, Glutarsäure, Adipinsäure, Pimelinsäure, Korksäure, Azelainsäure, Sebacinsäure oder dimerisierter oder trimerisierter Linolsäuren, cycloaliphatischen Polycarbonsäuren wie Tetrahydrophthalsäure, 4-Methyltetrahydrophthalsäure, Hexahydrophthalsäure und 4-Methylhexahydrophthalsäure und von aromatischen Polycarbonsäuren wie Phthalsäure, Isophthalsäure und Terephthalsäure ableiten. Weitere geeignete Polyglycidylester sind durch Polymerisation der Glycidylester von olefinisch ungesättigten Säuren, insbesondere von Glycidylarcrylat und Glycidylmethacrylat, erhältlich.

Ferner geeignet sind Polyglycidyl- und Poly(β-methylglycidyl)ether, wie sie durch Umsetzung einer mindestens zwei freie alkoholische und/oder phenolische Hydroxylgruppen pro Molekül enthaltenden Verbindung mit dem entsprechenden Epichlorhydrin unter alkalischen Bedingungen, oder auch in Gegenwart eines sauren Katalysators mit nachfolgender Alkalibehandlung erhältlich sind. Beispiele von Alkoholen und Phenolen für eine solche Umsetzung sind Ethylenglykol, Propandiol, Diethylenglykol, Poly(oxyethylen)glykole, Poly(oxypropylen)glykole, Poly(oxytetramethylen)glykole, Glycerin, 1,1,1-Trimethylolpropan, Pentaerythrit, Sorbit, Bis(4-hydroxy-cyclohexyl)-methan, 2,2-Bis(4-hydroxycyclohexyl)-propan, N,N-Bis(2-hydoxyethyl)anilin, p,p′-Bis(2-hydroxyethylamino)diphenylmethan, Bis(4-hydroxyphenyl)propan, sowie Novolake, wie sie durch Umsetzung von Aldehyden wie Formaldehyd oder Acetaldehyd, mit Phenolen hergestellt werden können.

Beispiele für Epoxidharze mit Gruppen der Formel VI, worin $R^3$ und $R^5$ zusammen eine $-CH_2CH_2-$Gruppe bedeuten, sind Bis-(2,3-epoxycyclopentyl)-ether oder 2,3-Epoxycyclopentylglycidylether.

Verwendbar sind auch Epoxidharze, in denen einige oder sämtliche Epoxidgruppen mittelständig sind, wie Vinylcyclohexendioxid, Dicyclopentadiendioxid, sowie epoxidierte Polybutadiene oder epoxidierte Copolymere des Butadiens mit Vinylmonomeren. Natürlich kann man auch Epoxidharzgemische verwenden.

Besonders bevorzugt verwendete Epoxidharze sind die Diglycidylether von zweiwertigen Phenolen und von zweiwertigen aliphatischen Alkoholen.

Gewünschtenfalls kann man das Epoxidharz auch in an sich bekannter Art einer Mischhärtung mit einem mehrwertigen Alkohol, insbesondere einem mit Molekulargewicht von über 1000 unterwerfen. Geeignete Alkohole dafür sind beispielsweise Poly(oxyethylen)glykole, Polyvinylalkohole, Hydroxypropylcellulose und Teilester der Cellulose.

Olefinisch ungesättigte Monomere a), die kationisch mit den erfindungsgemäßen Sulfoniumsalzen polymerisiert werden können, sind z.B. Styrol, α-Methylstyrol, Divinylbenzol, Vinylcyclohexan, 4-Vinylcyclohexen-1, N-Vinylcarbazol, Isopren, Butadien, sowie bevorzugt Vinylether wie Methylvinylether, Isobutylvinylether, 1,1,1-Trimethylolpropantrivinylether, Glycerintrivinylether, die Vinylether von Ethylenglykol und Polyethylenglykolen sowie cyclische Vinylether.

Die als Komponente a) bevorzugten Aminoplaste enthalten pro Molekül mindestens zwei an ein Amid- oder Thioamidstickstoffatom bzw. -atome gebundene Methylolgruppen, die auch verethert oder verestert sein können. Beispiele solcher Aminoplaste sind die N-Hydroxymethyl-, N-Methoxymethyl-, N-Butoxymethyl- und N-Acetoxymethylderivate von Harnstoff, Thioharnstoff oder cyclischen Harnstoffen, von Carbamaten und Dicarbamaten aliphatischer einwertiger und zweiwertiger Alkohole, sowie von Melamin, wie teilverethertes Hexamethylolmelamin oder von weiteren Polyamino-1,3-triazinen. Bevorzugte Aminoplaste sind die Kondensationsprodukte des Harnstoffs, Hydantoins oder Melamins mit Formaldehyd, z.B. ein Kondensationsprodukt aus Harnstoff mit 1,8 Mol Formaldehyd, sowie teilweise oder völlig veretherte Produkte solcher Kondensationsprodukte mit einem aliphatischen einwertigen Alkohol mit 1 bis 4 Kohlenstoffatomen, wie Hexamethoxymethylmelamin.

Als Phenoplaste werden die bekannten aus einem ein- oder mehrwertigen Phenol und einem Aldehyd wie Formaldehyd hergestellten Resole bevorzugt. Den erfindungsgemäßen härtbaren Mischungen können gegebenenfalls geeignete Zusätze wie Verdünnungsmittel, Verstärkungsmittel, Füllstoffe, Farbstoffe, Pigmente, Verarbeitungshilfsmittel oder andere übliche Zusatzstoffe zugegeben werden, deren Art und Menge dem Fachmann bekannt sind.

Die mit Hilfe der erfindungsgemäßen Sulfoniumsalze hergestellten härtbaren Harzzusammensetzungen können zur Beschleunigung der Härtung ferner als zusätzliche Komponente ein Oxidationsmittel aus der Klasse der Chinone und der organischen Peroxide enthalten. Geeignete Verbindungen sind beispielsweise Keton-Peroxide, Peroxysäuren, Aldehyd-Peroxide, Hydroperoxide, vor allem jedoch Alkylperoxide, Diacylperoxide und Alkylester von Persäuren wie z.B. Butylperoxypivalat, Benzoylperoxid, Di-tert.-butylperoxid, tert.-Butylhydroperoxid, Methylethylketon-Peroxid, m-Chlorperbenzoesäure. Beispiele für geeignete Chinone sind die ganz oder teilweise durch Chlor- oder Cyan-Gruppen substituierten Benzochinone wie Chloranil oder 2,3-Dichlor-5,6-Dicyanbenzochinon.

Die erfindungsgemäßen härtbaren Mischungen enthalten im allgemeinen 0,1 bis 15, vorzugsweise 0,5 bis 10 Gew.% der erfindungsgemäßen Sulfoniumsalze sowie gegebenenfalls 0,01 - 10, vorzugsweise 0,05 - 2 Gew.% der vorstehend genannten Oxidationsmittel, stets bezogen auf die Gesamtmenge der härtbaren Verbindungen a).

Die erfindungsgemäßen Mischungen können durch Erhitzen oder durch Bestrahlung mit aktinischem Licht der Wellenlänge 200 - 600 nm gehärtet werden, wobei das optimale Härtungsverfahren von den eingesetzten Komponenten der Mischungen sowie vom verwendungszweck derselben abhängt. Vorzugsweise enthalten die erfindungsgemäßen Zusammensetzungen auch einen Sensibilisator. Es wurde gefunden, daß die Einarbeitung geeigneter Sensibilisatoren die Härtungsgeschwindigkeit noch weiter steigert, was die Anwendung noch kürzerer Belichtungszeiten und/oder weniger starker Bestrahlungsquellen ermöglicht. Außerdem wird die Empfindlichkeit für sichtbares Licht erhöht. Geeignete Sensibilisatoren sind Acetophenonderivate wie Benzildimethylketal oder Benzoinether, Benzophenon oder dessen Derivate sowie Thioxanthon-Derivate wie 2-Methyl- oder 2-Isopropylthioxanthon. Weiter geeignete Sensibilisatoren sind polycyclische Aromaten, wie Anthracen, Phenanthren, Rubren, Perylen und Pyren. Vorzugsweise arbeitet man mit 0,1 - 2 Gew.% an Sensibilisatoren, bezogen auf die Gesamtmenge an Komponenten a).

Als aktinische Strahlungsquellen für die Photohärtung bei 200 - 600 nm Wellenlänge eignen sich die bekannten, wie Kohlebogenlampen, Quecksilberdampflampen, ultraviolettes Licht ausstrahlende Leuchtröhren, Argon- und Xenonglimmlampen und photographische Flutlampen. Die zur Belichtung erforderliche Zeit hängt unter anderem vom verwendeten polymerisierbaren Material, der Art der Lichtquelle und deren Abstand vom bestrahlten Material ab und ist vom Fachmann in einem Vortest leicht bestimmbar.

Soll die härtbare Zusammensetzung thermisch gehärtet werden, so wird sie in eine geeignete Form gebracht, z.B. als dünner Film ausgegossen. Zur Härtung wird das Harz auf Temperaturen zwischen 80 und 160°C, vorzugsweise zwischen 100 und 150°C, erhitzt.

Die erfindungsgemäßen Zusammensetzungen sind z.B. als Oberflächenbeschichtungen verwendbar und nach Aufbringen auf ein Substrat wie Stahl, Aluminium, Kupfer, Cadmium, Zink, Papier oder Holz durch Bestrahlen oder Erhitzen aushärtbar. Bestrahlt man durch eine Maske, so kann man dann die unbelichteten Schichtanteile durch Auswaschen entfernen. Die erfindungsgemäßen Mischungen sind besonders für die Herstellung von Druckplatten und gedruckten Schaltungen verwendbar, wobei die bekannten Methoden zur Herstellung von Druckplatten und gedruckten Schaltungen aus photopolymerisierbaren Zusammensetzungen angewandt werden können.

Die erfindungsgemäßen Mischungen sind auch als Klebstoffe, bei der Herstellung von faserverstärkten Verbundwerkstoffen, einschließlich Plattenpreßmassen, zur Herstellung von Kitten und Spachtelmassen oder für Tauchbeschichtungen verwendbar.

Eine erfindungsgemäße Mischung mit z.B. einem Epoxidharz oder Phenoplast und einer zur Polymerisation dieses Epoxidharzes oder Phenolplastes bei Belichtung der Zusammensetzung mit aktinischer Strahlung wirksamen Menge des erfindungsgemäßen Sulfoniumsalzes kann auch eine härtende Menge eines latenten Heißhärters für das Epoxidharz oder Phenoplast enthalten, wie Polycarbonsäureanhydride, Komplexe von Aminen, insbesondere primären oder tertiären aliphatischen Aminen mit Bortrifluorid oder Bortrichlorid. Latente Vernetzungsmittel für Resole sind unter anderem Hexamethylentetramin und Paraformaldehyd. Die für die Heißhärtung erforderliche Temperatur und Erhitzungsdauer sowie die Anteile an hitzeaktivierbarem Härter lassen sich leicht durch Vorversuche in bekannter Weise ermitteln.

Eine besondere Anwendungsform der erfindungsgemäßen Mischungen mit erfindungsgemäßen copolymerisierbaren Sulfoniumsalzen ist die als lichtempfindliches Aufzeichnungsmaterial für die Herstellung von Reliefbildern oder Resistmustern mit einer auf einem dimenionsstabilen Träger aufgebrachten lichtempfindlichen härtbaren Schicht.

Es hat sich gezeigt, daß sich die in erfindungsgemäßen Mischungen verwendeten Sulfoniumsalze hervorragend als thermisch und photochemisch aktivierbare Katalysatoren zur photochemischen Abspaltung von phenolischen Schutzgruppen und damit zur Löslichkeitsdifferenzierung eignen. Bezüglich weiterer Angaben zu dieser Verwendung der erfindungsgemäßen Mischungen für solche Aufzeichnungsmaterialien sei auf die DE-A-33 26 036, DE-A-32 31 147 und DE-A-32 31 145 verwiesen. Der Einsatz von erfindungsgemäßen Sulfoniumsalzen der Formel (I) verleiht solchen Aufzeichnungsmassen eine gute Lagerstabilität bei geringer Nachhärtungszeit und insbesondere eine gleichmäßige Verteilung des Initiators in der Schicht. So können sie beispielsweise mehrere Wochen bei 50°C gelagert werden, ohne daß sich dadurch die sehr guten Eigenschaften der Aufzeichnungsmaterialien und die hohe Qualität der hieraus hergestellten Reliefbilder oder Resistmuster verschlechtern.

Erfindungsgemäße lichtempfindliche Aufzeichnungsmaterialien eignen sich für die Herstellung von Hoch-, Tief-, Offset- oder Siebdruckformen, Photoresisten und Lötstoppmasken. Sie eignen sich auch für Schicht-

übertragungsmaterialien bei der Herstellung von Leiterplatten, gedruckten Schaltungen, integrierten Schaltungen etc. Die Herstellung der Reliefbilder oder Resistmuster mittels der erfindungsgemäßen Sulfoniumsalze enthaltenden Aufzeichnungsmaterialien kann wahlweise nach negativ arbeitenden oder positiv arbeitenden Verfahren erfolgen, wie sie an sich bekannt und beispielsweise in der DE-A 23 09 062 sowie den DE-A 32 31 144, DE-A 32 31 145 und DE-A 32 31 147 beschrieben sind.

Für alle in den folgenden Beispielen angegebenen Verbindungen wurde die Struktur durch korrekte $^1H$-NMR-, IR- und Massenspektren sowie durch übereinstimmende Elementaranalysen bestätigt.

Die in den Beispielen genannten Prozente sind Gewichtsprozente.

Beispiel 1

4-(N-(Methacryloylethyl)-carbamoyl)phenyl-dimethylsulfoniumhexafluoroarsenat

Zu der Lösung von 86 g 4-Hydroxyphenyldimethylsulfoniumhexafluoroarsenat in 870 g Toluol und 443 g Tetrahydrofuran wurden bei Raumtemperatur 42,5 g Isocyanatoethylmethacrylat zugetropft. Dann wurde bei einer Innentemperatur von 23 bis 26°C eine Lösung von 3 g Triethylamin in 89 g Tetrahydrofuran innerhalb von 10 Minuten zugesetzt und das Reaktionsgemisch über Nacht bei Raumtemperatur nachgerührt. Die ausgefallenden Kristalle wurden abgesaugt, mit Toluol gewaschen und aus Ethanol umkristallisiert. Ausbeute: 110 g (88 %) farblose Kristalle vom Schmp. 95 - 97°C.

Beispiel 2

4-(N-(Methacryloylethyl)-carbamoyl)-phenyl-dimethylsulfoniumhexafluorophosphat

Bei 20 bis 25°C wurde zu einem Gemisch aus 870 g Toluol, 443 g Tetrahydrofuran, 90 g 4-Hydroxyphenyldimethylsulfoniumhexafluorophosphat und 51 g Isocyanatoethylmethacrylat innerhalb von 10 Minuten eine Lösung von 3 g Triethylamin in 89 g Tetrahydrofuran so zugetropft, daß kein merklicher Temperaturanstieg erfolgte. Nach 12 Stunden wurde abgesaugt, mit Toluol gewaschen und aus Ethanol umkristallisiert. Ausbeute: 112 g (82 %) farblose Kristalle vom Schmp. 102 - 104°C.

Beispiele 3 bis 8

In Analogie zu den in den Beispielen 1 und 2 angegebenen Vorschriften wurden die folgenden Sulfoniumsalze hergestellt:

| Bsp.-Nr. | Verbindung | Anion | Ausbeute [%] |
|---|---|---|---|
| 3 | 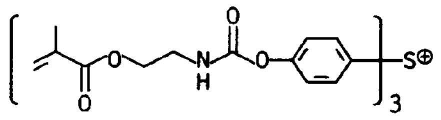 | $AsF_6^{\ominus}$ | 89 |
| 4 | 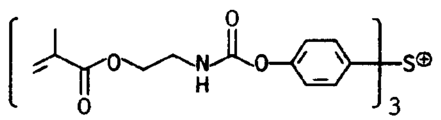 | $SbF_6^{\ominus}$ | 62 |
| 5 | 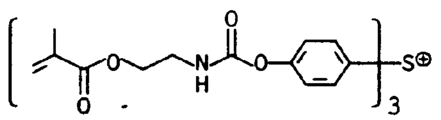 | $PF_6^{\ominus}$ | 77 |
| 6 | 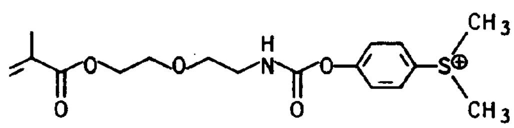 | $AsF_6^{\ominus}$ | 84 |
| 7 | 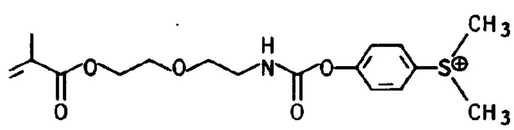 | $PF_6^{\ominus}$ | 80 |
| 8 | 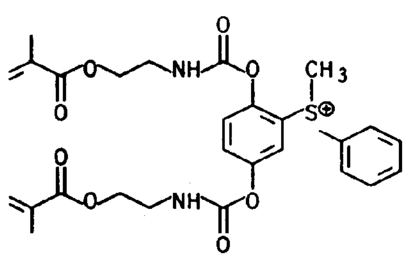 | $PF_6^{\ominus}$ | 73 |

Beispiel 9

4-(1-(Methacryloylethylcarbonato)naphthyl)tetrahydrothiopheniumchlorid

Die Mischung aus 533 g 4-(1-Hydroxynapthyl)tetrahydrothiopheniumchlorid und 540 g Hexamethyldisilazan wurde 8 Stunden bei 100°C unter Feuchtigkeitsausschluß gekocht. Nach dem Abkühlen auf Raumtemperatur wurde überschüssiges Silazan im Ölpumpenvakuum abdestilliert und der Rückstand (680 g) in 2300 g Acetonitril gelöst. Nach der Zugabe von 385 g 2-Chlorformylethylmethacrylat wurde 5 Stunden bei Raumtemperatur und dann 5 Stunden am Rückfluß erhitzt. Nach chromatograhischer Trennung (Kieselgel/Toluol) wurden 480 g (57 %) eines gelblichen Öles erhalten, das $^1H$-NMR- und $^{13}C$-NMR-spektroskopisch einheitlich war.

Beispiele 10 bis 17 (mit Vergleichsbeispielen)

Oberflächenbeschichtungen:

Jeweils 15 %igen acetonischen Lösungen von Bisphenol-A-diglycidylether wurden die nachstehenden Salze als Katalysatoren in Mengen zugesetzt, daß der Katalysatoranteil jeweils 3 %, bezogen auf den Bisphenol-A-diglycidylether betrug.

Die ohne Erwärmung hergestellten photoempfindlichen Mischungen wurden mit einem 80 µm-Rakel (effektive Schichtdicke ca. 50 µm) auf Glasplatten aufgezogen, vor der Belichtung 5 Minuten zur Entfernung des Acetons abgelüftet und unter Luft im Abstand von ca. 10 cm mit den jeweils angegebenen Bandgeschwindigkeiten an 2 Lampen mit 80 W/cm Leistung vorbeigeführt. Die Beurteilung der belichteten Schichten erfolgte sofort nach der Belichtung, nach 2 Stunden und nach 1 Tag nach folgenden Qualitätsmerkmalen:

| | |
|---|---|
| geliert | : nicht mehr fließfähig, klebrig, |
| fest | : Oberfläche klebfrei, jedoch nicht fingernagelhart, |
| durchgehärtet | : klebfrei, fingernagelhart. |

Tabelle 1: Härtung der Oberflächenbeschichtung

| Bsp.-Nr. | Verbindung | | Härtungsergebnis Bandgeschwindigkeit 3 m/min | | |
|---|---|---|---|---|---|
| | | | sofort | nach 2 Std. | nach 1 Tag |
| 10* | 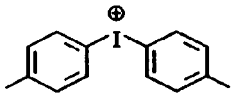 | $AsF_6^{\ominus}$ | durchgehärtet | durchgehärtet | durchgehärtet |
| 11* | 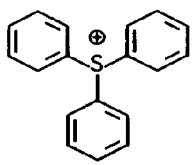 | $PF_6^{\ominus}$ | fest | durchgehärtet | durchgehärtet |
| 12 | 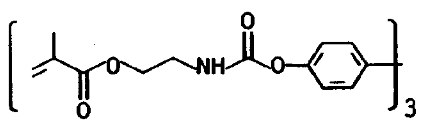 $S^{\oplus}$ $AsF_6^{\ominus}$ | | durchgehärtet | durchgehärtet | durchgehärtet |
| 13 | 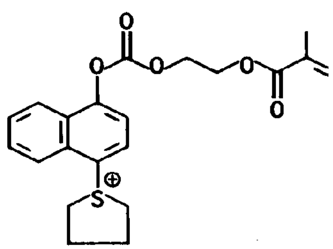 | $Cl^{\ominus}$ | durchgehärtet | durchgehärtet | durchgehärtet |

* Vergleichsbespiele

Tabelle 2: Härtung der Oberflächenbeschichtung

| Bsp.-Nr. | Verbindung | Härtungsergebnis Bandgeschwindigkeit 15 m/min sofort | nach 2 Std. | nach 1 Tag |
|---|---|---|---|---|
| 14* | 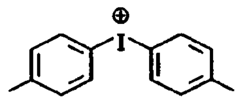 $AsF_6^{\ominus}$ | fest | fest | durchgehärtet |
| 15* | 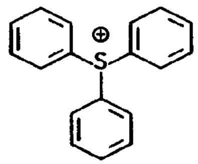 $PF_6^{\ominus}$ | geliert | fest | fest |
| 16 | 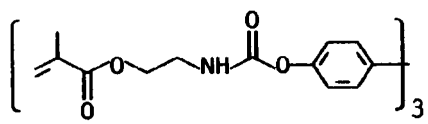 $S^{\oplus}$ $AsF_6^{\ominus}$ | fest | fest | durchgehärtet |
| 17 | 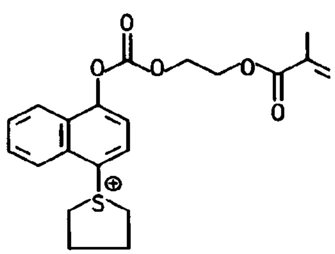 $Cl^{\ominus}$ | fest | fest | durchgehärtet |

* Vergleichsbespiele

Wie die in den Tabellen 1 und 2 angegebenen Ergebnisse zeigen, sind die erfindungsgemäßen Mischungen gemäß Beispielen 12, 13, 16 und 17 ebenso gut bzw. besser als die der Vergleichsbeispiele, die in den Fällen 10 und 14 jedoch ein toxisches Katalysatorsalz enthalten.

Mit Hilfe von ESCA-Messungen kann man zeigen, daß das Triarylsulfoniumhexafluorophosphat (Vergleichsbeispeile 11 und 15) in der Schicht einen Konzentrationsgradienten aufweist, der zum Trägermaterial hin größer wird und für die schlechtere Härtung verantwortlich ist.

## Patentansprüche

1. Strahlungsempfindliche, ethylenisch ungesättigte, copolymerisierbare, organische Verbindungen der allgemeinen Formel (I)

$$[(R)_a(R^1)_b(R^2)_c \overset{\oplus}{S}]\, A^{\ominus} \quad (I),$$

wobei

R einen, gegebenenfalls substituierten, einwertigen aromatischen organischen Rest bedeutet,

$R^1$ einen, gegebenenfalls substituierten, einwertigen organischen aliphatischen Rest aus der Gruppe der Alkyl-, Cycloalkyl- und substituierten Alkylreste bedeutet,

$R^2$ einen, gegebenenfalls substituierten, zwei- oder dreiwertigen aliphatischen oder aromatischen organischen Rest bedeutet, der eine heterocyclische oder kondensierte Ringstruktur bildet,

a eine ganze Zahl von 0 bis einschließlich 3 ist,

b eine ganze Zahl von 0 bis einschließlich 2 ist,

c eine ganze Zahl gleich 0 oder 1 ist,

wobei die Summe a+b+c=3 ist und

$A^{\ominus}$ für das Anion einer Säure steht

und mindestens einer der Reste R bis $R^2$ einen der Reste

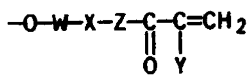

oder

$$-O-W-X-Z-CH=CH_2$$

gebunden enthält, worin

W für eine Einfachbindung, oder eine der Gruppen

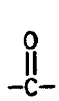 , 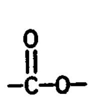 , 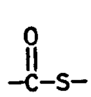 , 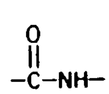 , 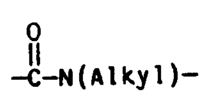 , 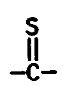 , 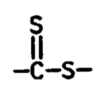 ,

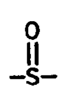 , 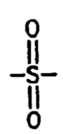 , 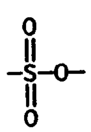 , 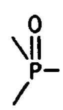 oder 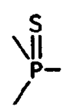 ,

X für einen zweiwertigen, gegebenenfalls substituierten, Alkylenrest $-(CH_2)_m-$, einen Rest

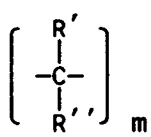 ,

mit m = 1 bis 10,

worin R′ und R″ untereinander gleich oder verschieden sind und für Aryl, $C_1$- bis $C_4$-Alkyl, H, COOH, $COOCH_3$ oder $COOC_2H_5$ stehen,

einen perfluorierten Alkylenrest $-(CF_2)_m-$ mit m = 1 bis 10, einen Oxaalkylenrest $-(CH_2)_n-O-(CH_2)_p-$ mit n = 1 bis 5 und p = 1 bis 5, einen perfluorierten Oxaalkylenrest $-(CF_2)_n-O-(CF_2)_p-$ mit n, p = 1 bis 5, oder einen, gegebenenfalls perfluorierten, Polyoxaalkylenrest mit 2 bis 20 Sauerstoffatomen, die miteinander über mindestens eine $-CH_2-$, $-CF_2-$ oder $-CH_2-CH(CH_3)$-Gruppe verbunden sind, für einen der Reste $-(CH_2)_m-O-CO-O-(CH_2)_n-$, $-(CH_2)_n-O-CO-NH-(CH_2)_m-$, $-(CH_2)_n-NH-CO-O-(CH_2)_m-$, $-(CH_2)_m-CO-O-(CH_2)_n-$ oder $-(CH_2)_m-O-CO-(CH_2)_n-$, mit m = 1 bis 10, n = 1 bis 10, einen gegebenenfalls mit Alkyl mit 1 bis 4 Kohlenstoffatomen, OH, $OCH_3$, $OC_2H_5$, SH, $SCH_3$, $SC_2H_5$, Cl, F, $N(Alkyl)_2$ oder $N(CH_3)C_6H_5$ in o-, m- und/oder p-Stellung substituierten, Phenylen-, oder einen Cycloalkylenrest mit 5 bis 10 Kohlenstoff-Atomen oder für einen (Bis)methylencycloalkylenrest mit 6 bis 12 Kohlenstoffatomen steht,

Y für H, Alkyl mit 1 bis 6 Kohlenstoffatomen oder Phenyl und

Z für O oder NY stehen.

2. Verfahren zur Herstellung von Verbindungen der allgemeinen Formel (I), dadurch gekennzeichnet, daß man eine Verbindung der Formel (II), (III) oder (IV)

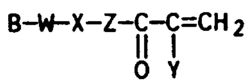
,
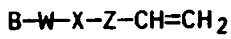
oder
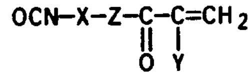

(II) (III) (IV)

worin

W, X, Y und Z die in Anspruch 1 genannte Bedeutung haben und

B für eine der Gruppen Tosylat, Alkoxy mit 1 bis 5 Kohlenstoffatomen, Halogen, Chlorcarbonyl, Imidazolyl, Pyrazolyl, Ammonium-, Phosphonium-, Sulfonium- oder Pyridiniumkation steht,

mit einer Verbindung der allgemeinen Formel (V)

$$[(R)_a(R^1)_b(R^2)_c \overset{\oplus}{S}]A^{\ominus} \quad (V),$$

wobei

R einen, gegebenenfalls substituierten, einwertigen aromatischen organischen Rest bedeutet,

$R^1$ einen, gegebenenfalls substituierten, einwertigen organischen aliphatischen Rest aus der Gruppe der Alkyl-, Cycloalkyl- und substituierten Alkylreste bedeutet,

$R^2$ einen gegebenenfalls substituierten zwei- oder dreiwertigen aliphatischen oder aromatischen organischen Rest bedeutet, der eine heterocyclische oder kondensierte Ringstruktur bildet,

a eine ganze Zahl von 0 bis einschließlich 3 ist,

b eine ganze Zahl von 0 bis einschließlich 2 ist,

c eine ganze Zahl gleich 0 oder 1 ist, wobei die Summe a+b+c=3 ist und

$A^{\ominus}$ für das Anion einer Säure steht

und mindestens einer der Reste R bis $R^2$ eine Hydroxylgruppe enthält, im äquimolaren Verhältnis oder entsprechend der Anzahl der Hydroxylgruppen in den Resten R bis $R^2$ mit dem Vielfachen davon, gegebenenfalls in Gegenwart eines inerten Lösungsmittels oder Lösungsmittelgemisches und eines basischen Katalysators, bei Temperaturen von 0 bis 100°C umsetzt.

3. Verfahren nach Anspruch 2, dadurch gekennzeichnet, daß es sich bei den Verbindungen der allgemeinen Formel (II), (III) und (IV) um ω-Chlorformylalkyl-(meth)-acrylate, ω-Chlorformylalkylvinylether bzw. ω-Isocyantoalkyl-(meth)-acrylate handelt.

4. Verfahren nach einem der Ansprüche 2 oder 3, dadurch gekennzeichnet, daß, bezogen auf die Anzahl der Hydroxylgruppen in den Resten R bis $R^2$ in der allgemeinen Formel (V), mindestens eine äquimolare Menge einer starken, nicht nucleophilen Base, vorzugsweise eines tertiären Amins, anwesend ist.

5. Verfahren nach einem der Ansprüche 2 bis 4, dadurch gekennzeichnet, daß die Reaktionstemperatur im Bereich von 10 bis 60°C liegt.

6. Verfahren nach einem der Ansprüche 2 bis 5, dadurch gekennzeichnet, daß insbesondere bei Verwendung der Isocyanate der Formel (IV) in einem inerten, wasserfreien Lösungsmittel, gegebenenfalls unter

Feuchtigkeitsausschluß gearbeitet wird.

7. Strahlungsempfindliches Gemisch, dadurch gekennzeichnet, daß es als Photoinitiator mindestens eine ethylenisch ungesättigte, copolymerisierbare, strahlungsempfindliche organische Verbindung nach Anspruch 1, gegebenenfalls im Gemisch mit weiteren reaktiven ethylenisch ungesättigten Verbindungen, Sensibilisatoren und weiteren üblichen Zusatzstoffen enthält.

8. Verwendung von copolymerisierbaren, ethylenisch ungesättigten, strahlungsempfindlichen organischen Verbindungen nach Anspruch 1 zur Herstellung polymerer, strahlungsempfindlicher Verbindungen.

9. Verwendung von strahlungsempfindlichen copolymerisierbaren, ethylenisch ungesättigten, organischen Verbindungen nach Anspruch 1 zur kationischen Polymerisation von Epoxidharzen.

10. Verwendung von strahlungsempfindlichen copolymerisierbaren, ethylenisch ungesättigten, organischen Verbindungen nach Anspruch 1 zur kationischen Polymerisation von Phenol-Formaldehydharzen.

11. Verwendung von strahlungsempfindlichen copolymerisierbaren, ethylenisch ungesättigten, organischen Verbindungen nach Anspruch 1 zur kationischen Polymerisation von Vinylethern.

12. Verwendung von strahlungsempfindlichen copolymerisierbaren, ethylenisch ungesättigten, organischen Verbindungen nach Anspruch 1 in positiv oder negativ arbeitenden Aufzeichnungsmaterialien zur Herstellung von Reliefbildern.

13. Verwendung von strahlungsempfindlichen, copolymerisierbaren organischen Verbindungen nach Anspruch 1, als Synthesezwischenprodukte bei der Herstellung von weitere funktionelle Gruppen enthaltenden Initiatoren sowie zur Herstellung von strahlungsreaktiven Systemen mit kovalent eingebundenen Photoinitiatoren.

14. Verwendung von strahlungsempfindlichen, ethylenisch ungesättigten, copolymerisierbaren Verbindungen nach Anspruch 1, worin mindestens einer der Reste R bis $R^2$ der allgemeinen Formel (I) den Rest -O-W-X-O-CH=$CH_2$ gebunden enthält, als reaktive Komponenten bei kationischen Polymerisationen zur Herstellung polymerer, strahlungsempfindlicher Verbindungen und als Comonomere in Hybrid-(Double-Cure)Systemen.

## Claims

1. A radiation-sensitive, ethylenically unsaturated, copolymerizable, organic compound of the formula (I)

$$[(R)_a(R^1)_b(R^2)_c \overset{\oplus}{S}]\, A^{\ominus} \quad (I),$$

(I)
where
R is an unsubstituted or substituted monovalent aromatic organic radical,
$R^1$ is an unsubstituted or substituted monovalent organic aliphatic radical from the group consisting of the alkyl, cycloalkyl and substituted alkyl radicals,
$R^2$ is an unsubstituted or substituted divalent or trivalent aliphatic or aromatic organic radical which forms a heterocylic or fused ring structure,
a is an integer from 0 up to and including 3,
b is an integer from 0 up to and including 2,
c is the integer 0 or 1,
the sum a+b+c being 3,
$A^{\ominus}$ is an anion of an acid and
at least one of the radicals R to $R^2$ contains one of the radicals

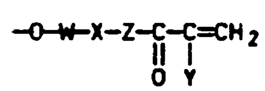

or

$-O-W-X-Z-CH=CH_2$

where

W is a single bond or one of the groups

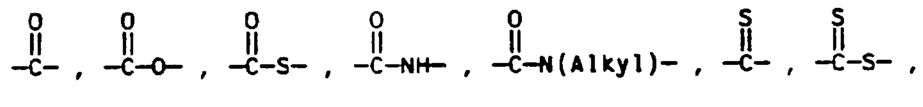

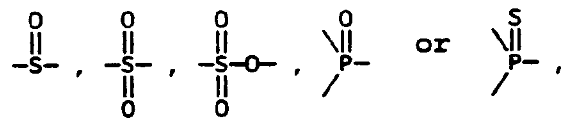

and

X is a divalent, unsubstituted or substituted alkylene radical $-(CH_2)_m-$, a radical

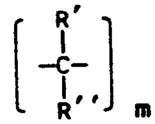

where m is from 1 to 10 and R′ and R″ are identical or different and are each aryl, $C_1$-$C_4$-alkyl, H, COOH, $COOCH_3$ or $COOC_2H_5$, or X is a perfluorinated alkylene radical $-(CF_2)_m-$, where m is from 1 to 10, an oxaalkylene radical $-(CH_2)_n-O-(CH_2)_p-$, where n and p are each from 1 to 5, a perfluorinated oxaalkylene radical $-(CF_2)_n-O-(CF_2)_p-$, where n and p are each from 1 to 5, or a polyoxaalkylene radical which may be perfluorinated and has from 2 to 20 oxygen atoms which are bonded to one another by at least one $-CH_2-$, $-CF_2-$ or $-CH_2-CH(CH_3)-$ group, or one of the radicals $-(CH_2)_m-O-CO-O-(CH_2)_n-$, $-(CH_2)_n-O-CO-NH-(CH_2)_m-$, $-(CH_2)_n-NH-CO-O-(CH_2)_m-$, $-(CH_2)_m-CO-O-(CH_2)_n-$ or $-(CH_2)_m-O-CO-(CH_2)_n-$, where m and n are each from 1 to 10, a phenylene radical which is unsubstituted or substituted by alkyl of 1 to 4 carbon atoms, OH, $OCH_3$, $OC_2H_5$, SH, $SCH_3$, $SC_2H_5$, Cl, F, $N(alkyl)_2$ or $N(CH_3)C_6H_5$ in the o-, m- and/or p-position, or a cycloalkylene radical of 5 to 10 carbon atoms, or a (bis)methylenecycloalkylene radical of 6 to 12 carbon atoms,

Y is H, alkyl of 1 to 6 carbon atoms or phenyl and Z is O or NY.

**2.** A process for the preparation of a compound of the formula (I), wherein a compound of the formula (II), (III) or (IV)

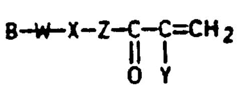 , 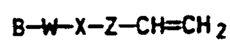 or 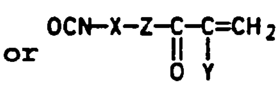

(II) (III) (IV)

where

W, X, Y and Z have the meanings stated in claim 1 and B is one of the groups tosylate, alkoxy of 1 to 5 carbon atoms, halogen, chlorocarbonyl, imidazolyl, pyrazolyl or an ammonium, phosphonium, sulfonium or pyridinium cation, is reacted with a compound of the formula (V)

$$[(R)_a(R^1)_b(R^2)_c\ \overset{\oplus}{S}\ ]A^{\ominus} \quad (V),$$

(V)

where

R is an unsubstituted or substituted monovalent aromatic organic radical,

$R^1$ is an unsubstituted or substituted monovalent organic aliphatic radical from the group consisting of the alkyl, cycloalkyl and substituted alkyl radicals,

$R^2$ is an unsubstituted or substituted divalent or trivalent aliphatic or aromatic organic radical which forms a heterocyclic or fused ring structure,

a is an integer from 0 up to and including 3,

b is an integer from 0 up to and including 2,
c is the integer 0 or 1,
the sum a+b+c being 3, and
$A^{\ominus}$ is an anion of an acid,
and at least one of the radicals R to $R^2$ contains a hydroxyl group, in an equimolar ratio or, depending on the number of hydroxyl groups in the radicals R to $R^2$, in a multiple thereof, in the presence or absence of an inert solvent or solvent mixture and of a basic catalyst, at from 0 to 100°C.

3. A process as claimed in claim 2, wherein the compounds of the formulae (II), (III) and (IV) are ω-chloroformylalkyl (meth)acrylates, ω-chloroformylalkyl vinyl ethers or ω-isocyanatoalkyl (meth)acrylates.

4. A process as claimed in claim 2 or 3, wherein at least an equimolar amount, based on the number of hydroxyl groups in the radicals R to $R^2$ in the formula (V), of a strong, nonnucleophilic base, preferably of a tertiary amine, is present.

5. A process as claimed in any of claims 2 to 4, wherein the reaction temperature is from 10 to 60°C.

6. A process as claimed in any of claims 2 to 5, wherein, particularly when the isocyanates of the formula (IV) are used, the reaction is carried out in an inert, anhydrous solvent, if necessary in the absence of moisture.

7. A radiation-sensitive mixture which contains, as the photoinitiator, at least one ethylenically unsaturated, copolymerizable, radiation-sensitive organic compound as claimed in claim 1, if necessary as a mixture with further reactive ethylenically unsaturated compounds, sensitizers and other conventional additives.

8. The use of a copolymerizable, ethylenically unsaturated, radiation-sensitive organic compound as claimed in claim 1 for the preparation of a polymeric, radiation-sensitive compound.

9. The use of a radiation-sensitive copolymerizable, ethylenically unsaturated, organic compound as claimed in claim 1 for the cationic polymerization of an epoxy resin.

10. The use of a radiation-sensitive copolymerizable, ethylenically unsaturated, organic compound as claimed in claim 1 for the cationic polymerization of a phenol/formaldehyde resin.

11. The use of a radiation-sensitive copolymerizable, ethylenically unsaturated, organic compound as claimed in claim 1 for the cationic polymerization of a vinyl ether.

12. The use of a radiation-sensitive copolymerizable, ethylenically unsaturated, organic compound as claimed in claim 1 in a positive-working or negative-working recording material for the production of relief images.

13. The use of a radiation-sensitive copolymerizable, organic compound as claimed in claim 1 as an intermediate in the synthesis of an initiator containing further functional groups and for the preparation of a radiation-reactive system containing covalently bonded photoinitiators.

14. The use of a radiation sensitive, ethylenically unsaturated, copolymerizable compound as claimed in claim 1, in which at least one of the radicals R to $R^2$ of the formula (I) contains a radical $-O-W-X-O-CH=CH_2$ in bonded form, as a reactive component in cationic polymerisation for the preparation of a polymeric, radiation-sensitive compound and as a comonomer in a hybrid (double-cure) system.

## Revendications

1. Composés organiques, copolymérisables, éthyléniquement insaturés, sensibles aux rayonnements, de la formule générale (I)

$$[(R)_a(R^1)_b(R^2)_c \overset{\oplus}{S}]A^{\ominus} \quad (I),$$

dans laquelle
R représente un radical organique, aromatique, monovalent, éventuellement substitué,
$R^1$ représente un radical aliphatique, organique, monovalent, éventuellement substitué, choisi parmi les radicaux alkyle, cycloalkyle et alkyle substitué,

$R^2$ représente un radical organique, aliphatique ou aromatique, di ou trivalent, éventuellement substitué, qui forme une structure hétérocyclique, ou cyclique condensée,

a représente un nombre entier de 0 à 3 inclusivement,

b représente un nombre entier de 0 à 2 inclusivement,

c représente un nombre entier égal à 0 ou à 1, où la somme a+b+c=3 et

$A^{\ominus}$ représente l'anion d'un acide et

au moins l'un des symboles R à $R^2$ contient, en liaison, l'un des groupes

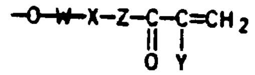

ou

$$-O-W-X-Z-CH=CH_2$$

dans lesquels

W représente une simple liaison ou l'un des radicaux qui suivent

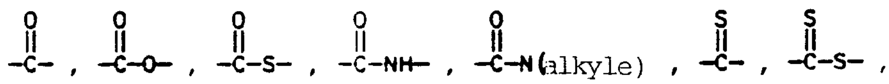

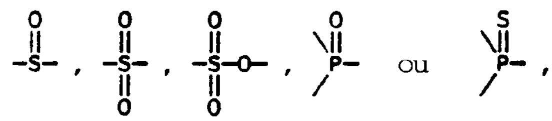

X représente un radical alkylène $-(CH_2)_m-$ divalent, éventuellement substitué, un radical

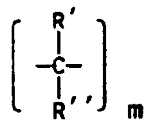

,

où m a une valeur qui varie de 1 à 10 et

R' et R'', mutuellement identiques ou différents, représentent chacun un radical aryle, alkyle en $C_1$ à $C_4$, un atome d'hydrogène, un radical COOH, $COOCH_3$ ou $COOC_2H_5$, un reste alkylène perfluoré $-(CF_2)_m-$ où m a une valeur qui varie de 1 à 10, un radical oxalkylène $-(CH_2)_n-O-(CH_2)_p-$ où n a une valeur qui varie de 1 à 5 et p a une valeur qui varie de 1 à 5, un radical oxalkylène perfluoré $-(CF_2)_n-O-(CF_2)_p-$ où n et p ont chacun une valeur qui varie de 1 à 5, ou un radical polyoxalkylène, éventuellement perfluoré, comportant de 2 à 20 atomes d'oxygène, qui sont liés les uns aux autres par au moins un radical $-CH_2-$, $-CF_2-$ ou $-CH_2-CH(CH_3)-$, l'un des radicaux $-(CH_2)_m-O-CO-O-(CH_2)_n-$, $-(CH_2)_n-O-CO-NH-(CH_2)_m-$, $-(CH_2)_n-NH-CO-O-(CH_2)_m-$, $-(CH_2)_m-CO-O-(CH_2)_n-$ ou $-(CH_2)_m-O-CO-(CH_2)_n-$, où m a une valeur qui varie de 1 à 10, n a une valeur qui varie de 1 à 10, un radical phénylène éventuellement substitué en positions ortho, méta et/ou para par des radicaux alkyle comportant de 1 à 4 atomes de carbone OH, $OCH_3$, $OC_2H_5$, SH, $SCH_3$, $SC_2H_5$, Cl, F, $N(alkyle)_2$ ou $N(CH_3)C_6H_5$, ou un radical cycloalkylène comportant de 5 à 10 atomes de carbone, ou un radical (bis)méthylènecycloalkylène comportant de 6 à 12 atomes de carbone,

Y représente un atome d'hydrogène, un radical alkyle comportant de 1 à 6 atomes de carbone, ou un radical phényle et

Z représente un atome d'oxygène ou NY.

**2.** Procédé de préparation de composés de la formule (I), caractérisé en ce que l'on fait réagir un composé de la formule (II), (III) ou (IV)

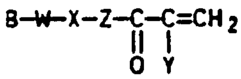 , 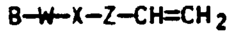 ou 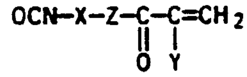

(II) (III) (IV)

où

W, X, Y et Z possèdent les significations qui leur ont été attribuées dans la revendication 1 et

B représente un atome d'halogène ou l'un des radicaux tosylate, alcoxy comportant de 1 à 5 atomes de carbone, chlorocarbonyle, imidazolyle, pyrazolyle, un cation ammonium, phosphonium, sulfonium ou pyridinium,

à des températures de 0 à 100°C éventuellement en présence d'un solvant ou d'un mélange de solvants inertes et d'un catalyseur basique,

avec un composé de la formule générale (V)

$$[(R)_a(R^1)_b(R^2)_c \overset{\oplus}{S}]\,A^{\ominus} \quad (V),$$

dans laquelle

R représente un radical organique, aromatique, monovalent, éventuellement substitué,

$R^1$ représente un radical aliphatique, organique, monovalent, éventuellement substitué, choisi parmi les radicaux alkyle, cycloalkyle et alkyle substitué,

$R^2$ représente un radical organique, aliphatique ou aromatique, di ou trivalent, éventuellement substitué, qui forme une structure hétérocyclique, ou cyclique condensée,

a représente un nombre entier de 0 à 3 inclusivement,

b représente un nombre entier de 0 à 2 inclusivement,

c représente un nombre entier égal à 0 ou à 1, où la somme a+b+c=3 et

$A^{\ominus}$ représente l'anion d'un acide et

et au moins l'un des groupes R à $R^2$ contient un radical hydroxyle, dans le rapport équimolaire ou dans un rapport multiple de celui-ci en correspondance au nombre des radicaux hydroxyle dans les groupes R à $R^2$.

**3.** Procédé suivant la revendication 2, caractérisé en ce qu'il s'agit, dans le cas des composés des formules générales (II), (III) et (IV) de (méth)acrylates d'ω-chloroformylalkyle, d'éthers ω-chloroformylalkylvinyliques ou de (méth)acrylates d'ω-isocyanatoalkyle.

**4.** Procédé suivant l'une quelconque des revendications 2 et 3, caractérisé en ce que, par rapport au nombre des radicaux hydroxyle dans les groupes R à $R^2$ de la formule générale (V), au moins une proportion équimolaire d'une base forte, non nucléophile, de préférence une amine tertiaire, est présente.

**5.** Procédé suivant l'une quelconque des revendications 2 à 4, caractérisé en ce que la température réactionnelle varie de 10 à 60°C.

**6.** Procédé suivant l'une quelconque des revendications 2 à 5, caractérisé en ce que, plus particulièrement lors de l'emploi des isocyanates de la formule (IV), on travaille dans un solvant inerte et dépourvu d'eau, éventuellement sous exclusion d'humidité.

**7.** Mélange sensible aux rayonnements, caractérisé en ce qu'il contient, à titre de photoamorceur, au moins un composé organique, sensible aux rayonnements, copolymérisable et éthyléniquement insaturé, suivant la revendication 1, éventuellement en mélange à d'autres composés éthyléniquement insaturés et réactifs, des sensibilisateurs et d'autres additifs usuels.

**8.** Utilisation de composés organiques, sensibles aux rayonnements, éthyléniquement insaturés, copolymérisables, suivant la revendication 1, pour la fabrication de composés polymériques, sensibles aux rayonnements.

**9.** Utilisation de composés organiques, éthyléniquement insaturés, copolymérisables, sensibles aux rayonnements, suivant la revendication 1 pour la polymérisation cationique de résines époxydes.

**10.** Utilisation de composés organiques, éthyléniquement insaturés, copolymérisables, sensibles aux rayonnements, suivant la revendication 1 pour la polymérisation cationique de résines de phénol et de formaldéhyde.

**11.** Utilisation de composés organiques, éthyléniquement insaturés, copolymérisables, sensibles aux rayonnements, suivant la revendication 1 pour la polymérisation cationique d'éthers vinyliques.

**12.** Utilisation de composés organiques, éthyléniquement insaturés, copolymérisables, sensibles aux rayonnements, suivant la revendication 1 dans des matériaux d'enregistrement travaillant en positif ou en négatif pour la réalisation d'images en relief.

**13.** Utilisation de composés organiques, copolymérisables, sensibles aux rayonnements, suivant la revendication 1, comme produits intermédiaires pour la synthèse, lors de la préparation d'autres amorceurs contenant des radicaux fonctionnels, en vue de la réalisation de systèmes réactifs par irradiation avec des photoamorceurs liés par covalence.

**14.** Utilisation de composés polymérisables, éthyléniquement insaturés, sensibles aux rayonnements, suivant la revendication 1, où au moins l'un des groupes R à $R^2$ de la formule générale (I) contient le radical $-O-W-X-O-CH=CH_2$ en liaison, à titre de composants réactifs au cours de polymérisations cationiques pour la préparation de composés polymériques sensibles aux rayonnements et à titre de comonomères dans des systèmes hybrides-(double-cure).